Europäisches Patentamt

European Patent Office

Office européen des brevets

(19)

(11) Veröffentlichungsnummer: **0 243 655 B1**

## (12) EUROPÄISCHE PATENTSCHRIFT

(45) Veröffentlichungstag der Patentschrift: **09.09.92**

(51) Int. Cl.⁵: **G01N 33/543**, //G01N33/577

(21) Anmeldenummer: **87104034.1**

(22) Anmeldetag: **19.03.87**

Die Akte enthält technische Angaben, die nach dem Eingang der Anmeldung eingereicht wurden und die nicht in dieser Patentschrift enthalten sind.

(54) **Verfahren und Reagenz zur Bestimmung eines Reaktionspartners einer immunologischen Reaktion.**

(30) Priorität: **19.03.86 DE 3609217**

(43) Veröffentlichungstag der Anmeldung:
**04.11.87 Patentblatt 87/45**

(45) Bekanntmachung des Hinweises auf die Patenterteilung:
**09.09.92 Patentblatt 92/37**

(84) Benannte Vertragsstaaten:
**AT BE CH DE ES FR GB GR IT LI LU NL SE**

(56) Entgegenhaltungen:
**EP-A- 0 093 613**
**EP-A- 0 098 590**
**WO-A-86/07633**
**FR-A- 2 410 278**
**US-A- 4 558 013**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Kaspar, Klaus Peter, Dr.**
**Gröberweg 1**
**W-8132 Tutzing(DE)**

(74) Vertreter: **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach 86 08 20**
**W-8000 München 86(DE)**

**Beschreibung**

Die Erfindung betrifft ein Verfahren zur Bestimmung eines Reaktionspartners einer immunologischen Reaktion nach dem Prinzip des Immunoassays, wobei der zu bestimmende Reaktionspartner mit einem markierten spezifischen Rezeptor $R_1$ und mit mindestens einem unmarkierten Rezeptor $R_2$ in Kontakt gebracht wird und wobei einer der unmarkierten Rezeptoren $R_2$ über eine bindefähige Substanz $R_3$ an eine Festphase gebunden ist.

Zur Bestimmung eines Reaktionspartners einer immunologischen Reaktion, im folgenden auch als Analyt bezeichnet, werden häufig Immunoassays nach dem Sandwich-Prinzip verwendet. Dabei wird ein Reaktionspartner mit einem für ihn spezifischen Rezeptor, der in an sich bekannter Weise markiert ist, umgesetzt und der Komplex aus Reaktionspartner und markiertem Rezeptor mit einem weiteren Rezeptor, der gegen den zu bestimmenden Reaktionspartner oder den Komplex gerichtet ist und an einer Festphase fixiert ist, umgesetzt. Für dieses Prinzip gibt es noch weitere Variationen, wobei die Reihenfolge dieser Reaktionsschritte beliebig variiert werden kann. So kann der Reaktionspartner mit einem löslichen unmarkierten und einem löslichen markierten Rezeptor, die jeweils gegen den zu bestimmenden Reaktionspartner (Analyten) gerichtet sind, umgesetzt werden und der Komplex aus den drei Bestandteilen dann mit einem unlöslichen Rezeptor, d. h. einem an eine Festphase gebundenen Rezeptor, der gegen den unmarkierten Rezeptor gerichtet ist, umgesetzt werden. Die Umsetzung mit allen drei Rezeptoren kann gleichzeitig erfolgen oder aber in Stufen. Bei allen diesen Verfahren wird die Menge an an die Festphase gebundenem markiertem Rezeptor oder an in der Lösung vorliegendem, nicht gebundenem markiertem Rezeptor über geeignete Meßverfahren bestimmt und zu der Menge an zu bestimmendem Reaktionspartner in Beziehung gesetzt.

Als Markierung werden beispielsweise radioaktive Substanzen, Enzyme, fluoreszierende oder farbige Substanzen verwendet. Der Gehalt an markiertem Rezeptor kann dann über die Veränderung der Fluoreszenzeigenschaft, Entwicklung eines farbigen Reaktionsproduktes oder Verteilung von radioaktiven Substanzen zwischen fester und flüssiger Phase bestimmt werden. Dazu kann aus der Änderung des Meßsignals entweder, wenn ausreichende theoretische Kenntnisse über den Zusammenhang zwischen Meßsignal und Gehalt bestehen, direkt der Gehalt des gesuchten Reaktionspartners berechnet werden oder es wird anhand einer Eichkurve einem Meßsignal ein Gehalt zugeordnet. Wesentlich bei beiden Verfahren ist, daß jeder Änderung des Meßsignals ein Gehalt an Reaktionspartner zugeordnet wird.

Es tritt nun aber bei dieser Art von Immunoassays das Problem auf, daß es zu unspezifischen Anlagerungen an die Festphase kommt. Diese Anlagerungen werden durch probenspezifische Einflüsse bedingt und werden als Matrixeffekt bezeichnet. Da auch markierte Rezeptoren unspezifisch an die Festphase angelagert werden, kommt es zu einer Verfälschung der Meßsignale und damit der Meßergebnisse. Um diesen Fehler zu berichtigen, muß in solchen Fällen ein Probenleerwert ermittelt werden. Die Ermittlung des Problenleerwertes führte jedoch bisher regelmäßig dann zu Schwierigkeiten, wenn gesichert analytfreie Proben nicht erhältlich sind oder wenn keine absolut messende Referenzmethode zur Feststellung der An- bzw. Abwesenheit des Analyten in der Probe existiert. In diesen Fällen wäre es für die korrekte Ermittlung des Probenleerwertes erforderlich, daß sich sowohl die Probe als auch der Standard identisch verhaltne. Gerade bei den immunologischen Bestimmungsmethoden ist dies jedoch nur sicher möglich, wenn jeweils dasselbe Untersuchungsmaterial verwendet wird. Für die Analytsbestimmung z.B. in Seren wurde deshalb schon vorgeschlagen, zur Standardherstellung Serumpools zu verwenden, aus denen der Analytgehalt mit chemischen, physikalischen oder immunologischen Methoden entfernt wurde. Nachteil dieses Verfahrens ist es jedoch, daß nicht kontrolliert werden kann, ob die Analytentfernung vollständig gelungen ist, da ja keine Meßwerte für eine Nullprobe zur Verfügung stehen. Andererseits ist aber selbst bei vollständiger Analytentfernung mit einer Veränderung des Probenleerwerts zu rechnen, da die Entfernung des Analytrestgehaltes eine Veränderung der Matrix bewirken kann, die wiederum den Eichkurvennullpunkt und die Eichkurvensteilheit beeinflussen kann. Ohne die Kenntnis des nach der Behandlung möglicherweise veränderten Probenleerwertes ist eine exakte Bestimmung des Analytrestgehaltes auch nicht möglich. Außerdem muß mit größeren Leerwertschwankungen bei Einsatz von Probenmaterialien mit stark schwankender Zusammensetzung, z. B. bei Verwendung von Gewebehomogenisaten gerechnet werden.

Weiterhin wurde vorgeschlagen, ein künstlich zusammengestelltes Material wie Pufferlösungen, Rinderserumalbuminlösungen etz. zu verwenden.

Solche Lösungen zeigen aber oft ein vom zu untersuchenden Probengut unterschiedliches Verhalten und führen deshalb zu Verfälschungen (I.Marschner et al, J. Clin.Chem.Clin.Biochem,1976,345-351).

Zudem ist bisher kein Verfahren bekannt, welches z. B. serumspezifische Probenleherwerte in unterschiedlichen Humanserumproben messen kann. Die Folge ist, daß für alle Proben ein gemeinsamer hypothetischer Leerwert angenommen werden muß. Damit wird zwangsläufig jeder Signalunterschied gegenüber diesem Leerwert einem Analytgehalt zugeschrieben, obwohl das Signal genausogut durch spezifische Probeneigenschaften verursacht sein kann.

Bisher gibt es noch kein Verfahren, mit dem es möglich ist, für die Bestimmng von physiologisch vorkommenden Analyten eine analytfreie Matrix zur Standardproduktion herzustellen, ohne daß eine potentielle Verfälschung der Eichkurvensteilheit oder des Eichkurvennullpunktes in Kauf genommen werden muß.

Dies bedeutet, daß eine Eichkurve mit korrekter Steigung nur bei Verwendung von Matrixmaterial gewährleistet ist, das mit dem Untersuchungsmaterial identisch ist. Wenn aber das gewählte Matrixmaterial selbst einen unbekannten Analytgehalt besitzt, kann der Nullpunkt der Eichkurve nicht bestimmt werden. Ohne die Kenntnis von der Lage des Eichkurvennullpunktes kann zwar die Steigung, nicht aber der Verlauf der Eichkurve bestimmt werden.

Ziel der Erfindung war es daher, ein Verfahren zur Verfügung zu stellen, mit Hilfe dessen es möglich ist, den Probenleerwert in derselben Matrix zu bestimmen, die auch für die Bestimmung des Analyten verwendet wird. Weiterhin war es Ziel der Erfindung, ein Verfahren zu schaffen, mit Hilfe dessen reproduzierbare Eichkurven und damit genaue Meßwerte erhalten werden können.

Dieses Ziel wird erreicht durch ein Verfahren zur Bestimmung eines Reaktionspartners einer immunologischen Reaktion nach dem Prinzip des Immunoassays, wobei der zu bestimmende Reaktionspartner mit einem markierten spezifischen Rezeptor $R_1$ und mindestens einem unmarkierten Rezeptor $R_2$ in Kontakt gebracht wird und wobei einer der unmarkierten Rezeptoren $R_2$ aan eine Festphase über eine bindefähige Substanz $R_3$ gebunden ist, das dadurch gekennzeichnet ist, daß zur Bestimmung des Probenleerwertes der unmarkierte Rezeptor $R_2$, welcher von dem an die Festphase fixierten Rezeptor gebunden wird, durch einen anderen unmarkierten Rezeptor $R_2'$, der mit dem anderen Reaktionspartner von $R_2$ nicht reagiert, ersetzt wird.

Bei dem erfindungsgemäßen Verfahren kann zur Bestimmung des Probenleerwertes dieselbe Matrix und dieselbe Festphase, einschließlich $R_3$, wie für die Bestimmung des Analyten verwendet werden. Auf eine Behandlung der Matrix zur Beseitigung etwa vorhandenen zu bestimmenden Reaktionspartners kann verzichtet werden. Auf diese Weise ist gewährleistet, daß Leerwert und Analyse im gewählten Untersuchungssystem ein gleichartiges Verhalten zeigen.

Zur Bestimmung eines Reaktionspartners einer immunologischen Reaktion, des Analyten, wird in an sich bekannter Weise der Analytgehalt einer Probe bestimmt, indem die Probe mit einem markierten, für den Reaktionspartner spezifischen Rezeptor $R_1$ und mindestens einem unmarkierten Rezeptor $R_2$ inkubiert wird. $R_2$ hat zwei Reaktionspartner, nämlich $R_3$ und entweder den Analyten selbst oder einen weiteren, seinerseits mit dem Analyten bindefähigen Rezeptor $R_{2a}$. Außerdem wird zur Bestimmung des Probenleerwerts eine Probe desselben Ursprungs ebenfalls mit Rezeptor $R_1$ inkubiert. Statt $R_2$ wird jedoch Rezeptor $R_2'$, der mit dem weiteren Reaktionspartner von $R_2$ nicht reagiert, zugefügt. Die Bestimmung selbst sowie die Probenleerwertbestimmung kann beispielsweise so erfolgen, daß zunächst $R_2$ bzw. $R_2'$ zugegeben werden, nach Inkubation und Waschschritt Probe zugegeben wird und nach weiterer Inkubation und Waschschritt $R_1$ zugegeben wird. Anschließend wird inkubiert, gegebenenfalls gewaschen und die Bestimmung der Markierung in der flüssigen oder festen Phase durchgeführt. Diese Reaktionsschritte können jedoch auch teilweise oder vollständig simultan oder in umgekehrter Reihenfolge durchgeführt werden. Hierbei muß jedoch darauf geachtet werden, daß $R_3$ im Überschuß gegen $R_2$ vorliegt, damit der zu bestimmende Reaktionspartner vollständig über $R_2$ an $R_3$ gebunden wird. Auf diese Weise kommt es in beiden Ansätzen zu den für die jeweilige Matrix typischen unspezifischen Anlagerungen, insbesondere des markierten Rezeptors $R_1$ an die Festphase. In dem Ansatz für den Probenleerwert findet jedoch die spezifische Reaktion nicht statt. Man erhält dann für jeden Ansatz ein Meßsignal, wobei das Signal, das durch unspezifische, sogenannte Matrixeffekte erzeugt wird, in beiden Ansätzen gleich ist, während für den Ansatz zur Bestimmung des Analyten das Signal um den Betrag größer ist, der dem Gehalt an Analyt entspricht. Mißt man nun eine Probe mit unbekanntem Analytgehalt gegen diesen Probenleerwert, so kann die Differenz der beiden Meßsignale der Menge an markiertem Rezeptor $R_1$ zugeordnet werden, die spezifisch von der an der Festphase fixierten bindefähigen Substanz $R_3$ gebunden wird. Man kann also durch den Vergleich der Meßsignale, welche bei Probenleerwertmessung und Analytmessung erhalten werden, unterscheiden zwischen dem Anteil der auf unspezifische Anlagerungen zurückzuführen ist und dem Anteil, der tatsächlich von dem zu bestimmenden Reaktionspartner erzeugt wird.

Als Rezeptoren werden im Rahmen der Erfindung vorzugsweise bindefähige komplette Antikörper Antikörperfragmente oder Konjugate von Antikörpern oder Antikörperfragmenten mit Haptenen verwendet.

Als Rezeptor $R_1$ wird ein markierter Rezeptor verwendet, der spezifisch für den Analyten ist, d. h. nur mit diesem bindet. Rezeptor $R_1$, der in bekannte Menge eingesetzt wird, ist in der dem Fachmann bekannten Weise markiert. Bevorzugt erfolgt die Markierung durch Kupplung mit einem Enzym, einer fluoreszierenden, chemilumineszierenden oder radioaktiven Subztanz. Verfahren zur Markierung von derartigen Rezeptoren sind dem Fachmann bekannt, z.B. aus Clin. Chim. Acta 81 (1977) 1-40, und bedürfen hier keiner weiteren Erläuterung.

Die Rezeptoren $R_2$ und $R_2'$ und gegebenenfalls $R_{2a}$ können Antikörper, Antikörperfragmente oder Derivate hiervon sein. $R_{2a}$ kann auch ein Bindepartner eines Antikörpers sein, beispielsweise wenn der Analyt selbst ein Antikörper ist. Als Derivate sind beispielsweise Antikörper oder Fragmente geeignet, die an einen Bindungspartner eines miteinander bindefähigen Substanzenpaares gekoppelt sind. In diesem Fall ist dann $R_3$ der andere Partner dieses Substanzenpaares. Als geeignete Substanzpaare sind z.B. Antigen/Hapten-Antikörper, Protein A-Immunglobulin G, Avidin-Biotin, Concanavalin A-Lectin, DNA-DNA-(Hybrid) zu nennen.

Als $R_2$ und $R_2'$ werden z. B. Antikörper oder Fragmente verwendet, welche an ein Hapten gekoppelt sind. In diesem Fall ist $R_3$ ein gegen das Hapten gerichteter Antikörper. Dabei muß vorzugsweise sichergestellt sein, daß die Rezeptoren $R_2$ und $R_2'$ annähernd gleich an die bindefähige Substanz $R_3$ binden. Bei Verwendung von haptenisierten Antikörpern oder Fragmenten sollte deshalb der Haptenisierungsgrad von $R_2$ und $R_2'$ vergleichbar bzw. identisch sein.

Als Rezeptor $R_2'$ , der bei dem Probenleerwert gegen den Rezeptor $R_2$ ausgetauscht wird, wird ein Rezeptor verwendet, der mit dem Analyten nicht reagieren kann. Dieser Rezeptor $R_2'$ soll keinen Einfluß auf die nichtspezifische Bindung des markierten Rezeptors $R_1$ an die Festphase haben, bzw. keinen Unterschied in der nichtspezifischen Bindung des markierten Rezeptors $R_1$ verursachen. Die Eignung eines Rezeptors $R_2'$ für ein bestimmtes System kann überprüft werden, indem man den markierten Rezeptor $R_1$ gegen einen gleichartig markierten aber für den Test nicht spezifischen Rezeptor $R_1'$ austauscht und anschließend die Meßsignale bei Abwesenheit und Anwesenheit des spezifischen Rezeptors $R_2$ bzw. nach Austausch von $R_2$ gegen einen Rezeptor $R_2'$ vergleicht. Sind die Meßsignale im Rahmen der Fehlergrenze gleich, so ist Rezeptor $R_2'$ für das erfindungsgemäße Verfahren geeignet.

Der Rezeptor $R_2$ bzw. der Rezeptor $R_2'$ wird über eine bindefähige Substanz $R_3$ an die Festphase gebunden. Geeignete bindefähige Substanzen $R_3$ sind beispielsweise Antikörper oder Antikörperfragmente sowie Bindungspartner der oben genannten Substanzpaare. Wenn $R_2$ und $R_2'$ Antikörper der Klasse IgG sind, kann $R_3$ beispielsweise Protein A oder ein Anti-Immunglobulin-Antikörper sein. Sind $R_2$ und $R_2'$ haptenisierte Antikörper, kann $R_3$ beispielsweise ein gegen das Hapten gerichteter Antikörper sein. Wird als $R_3$ ein Anti-Immunoglobulin-Antikörper verwendet, dann muß sichergestellt werden, daß $R_1$ nicht an $R_3$ bindet. Dies kann beispielsweise dadurch erreicht werden, daß $R_2$ und $R_1$ von verschiedenen nicht kreuzreagierenden Spezies sind oder daß als $R_3$ ein anderes Antikörperfragment als für $R_1$ verwendet wird. Beispielsweise ist dabei $R_3$ ein Anti-Fc$\gamma$-Antikörper, während $R_1$ ein Antikörperfragment wie Fab, Fab' oder F(ab')$_2$ ist.

Besonders bevorzugt wird als $R_3$ ein Antikörper verwendet, welcher nur mit einem Teil des Rezeptors $R_2$ bzw. $R_2'$ bindefähig ist, besonders bevorzugt ein Antikörper, der mit dem Fc-Teil des Rezeptors $R_2$ bzw. $R_2'$ bindefähig ist. Alternativ läßt sich als $R_3$ auch ein Antikörper verwenden, der mit dem Haptenteil von $R_2$ bzw. $R_2'$ oder dem Fab-Teil oder mit dem gesamten Rezeptor $R_2$ bzw. $R_2'$ spezifisch bindefähig ist. Die Bindung der in fester Phase vorliegenden bindefähigen Substanz $R_3$ an ein unlösliches Trägermaterial kann nach den üblichen, dem Fachmann bekannten Methoden zur Fixierung biologisch aktiver Proteine an feste Trägersubstanzen vorgenommen werden. Sowohl eine kovalente als auch eine adsorptive Bindung ist geeignet. Bevorzugt wird jedoch, wegen der hierbei erzielbaren höheren Ausbeute und der vereinfachten Arbeitsweise, eine lediglich adsorptive Bindung, beispielsweise an Kunststoff. Als besonders geeignet erwiesen sich dabei Reagenzgläschen aus Polystyrol und ähnlichen Kunststoffen, die adsorptiv an der Innenoberfläche mit $R_3$ beschichtet sind. Darüberhinaus gelten insbesondere für die Auswahl der Rezeptoren und der bindefähigen Substanz $R_3$ sowie für die Durchführung des Bestimmungsverfahrens die in der EP 0 098 590 enthaltenen Ausführungen.

Die verwendeten Rezeptoren können sowohl polyklonal als auch monoklonal sein. Ist der im Probenleerwert zu ersetzende Rezeptor $R_2$ monoklonal, so ist es bevorzugt, daß der ihn ersetzende Rezeptor $R_2'$ ebenfalls monoklonal und von derselben Spezies ist. Besonders bevorzugt gehört er dann derselben Subklasse an. Ist der Rezeptor $R_2$ polyklonal, so ist der unspezifische Rezeptor $R_2'$ bevorzugt ebenfalls polyklonal und von derselben Spezies. Besonders bevorzugt ist es dann, daß die Gesamtzusammensetzung von $R_2$ und $R_2'$ bezüglich der Subklassen möglichst ähnlich ist.

Mit dem erfindungsgemäßen Verfahren ist es auch möglich, eine Eichkurve für einen zu bestimmenden Reaktionspartner aufzustellen, deren Nullpunkt und Steigung exakt bestimmt werden können. Dazu wird eine Probe mit unbekanntem Gehalt an Analyt mit bekannten Mengen an Analyt aufgestockt. Für verschiedene Analytzugaben wird dann das Meßsignal bestimmt. Gleichzeitig wird in demselben System ein Probenleerwert bestimmt. Aus den erhaltenen Werten kann dann die Eichkurve erstellt werden, deren Steigung nicht durch Matrixeffekte oder sonstige Effekte verfälscht wird. Auf diese Weise wird eine genaue reproduzierbare Eichkurve erhalten. Mit dieser exakten Eichkurve können dann über die erhaltenen Meßsignale für eine Analytprobe auch die Konzentrationen des Analyten genau bestimmt werden.

Ein weiterer Gegenstand der vorliegenden Erfindung ist ein Reagenz zur Bestimmung eines Reaktionspartners einer immunologischen Reaktion, das dadurch gekennzeichnet ist, daß es eine an eine Festphase gebundene bindefähige Substanz $R_3$, mindestens einen mit $R_3$ und einem weiteren Reaktionspartner bindefähigen unmarkierten Rezeptor $R_2$ und einen markierten spezifischen Rezeptor $R_1$ enthält und physikalisch getrennt davon eine weitere Menge der an eine Festphase gebundenen bindefähigen Substanz $R_3$, einen unmarkierten Rezptor $R_2'$, der mit dem weiteren Reaktionspartner von $R_2$ nicht reagiert und eine weitere Menge an markiertem spezifischen Rezeptor $R_1$ enthält.

Bevorzugt liegt das Reagenz in Form zweier Reaktionsbehälter vor, die jeweils dieselbe Festphase und den denselben an die Festphase gebundenen Rezeptor $R_3$ enthalten, wobei der Rezeptor $R_3$ ein Anti-Ig-Antikörper ist. Besonders bevorzugt trägt der Rezeptor $R_3$ bereits $R_2$, bzw. $R_2'$. Alternativ liegen $R_1$ und $R_2$ bzw. $R_2'$ vorgemischt und $R_3$ getrennt davon vor. Alle Rezeptoren $R_2$ und $R_1$ können trocken, gelöst oder auf einem festen Träger ablösbar aufgebracht vorliegen.

In einer anderen bevorzugten Ausführungsform, in der die Rezeptoren $R_2$, bzw. $R_2'$ haptenisiert sind, sind diese beiden Rezeptoren bereits an die Festphase über einen, gegen das Hapten gerichteten Antikörper $R_3$ gebunden.

Erfindungsgemäß werden ein Verfahren und ein Reagenz zur Verfügung gestellt, die es ermöglichen, zur Bestimmung eines Reaktionspartners einer immunologischen Reaktion eine Eichkurve aufzustellen, deren Nullpunkt genau angegeben werden kann und deren Steigung nicht verfälscht wird. Ein weiterer Vorteil des erfindungsgemäßen Verfahrens besteht darin, daß bei einer parallelen Messung von Probe und Probenleerwert die Differenz des Meßsignals dem Gehalt an zu untersuchendem Reaktionspartner zugeordnet werden kann, da durch die Messung des Probenleerwerts unspezifische Anlagerungen erkannt werden können. Auf diese Weise ist es möglich, sehr sichere und korrekte Messungen durchzuführen.

Die Erfindung soll noch an Beispielen erläutert werden.

## Beispiel 1

Bestimmung von Thyreotropin (TSH) in Humanseren

1. Herstellung der Reagenzlösungen

1.1 Puffer I:

50 mmol/l Kalium-Phosphat-Puffer, pH 6,0, hergestellt durch Mischung von 50 mmol/l $K_2HPO_4$-Lösung und 50 mmol/l $KH_2PO_4$-Lösung bis zum Erreichen des pH-Wertes 6,0.

1.2 Puffer II:

Puffer II wird wie Puffer I hergestellt mit dem Unterschied, daß als pH-Wert der pH 7,5 eingestellt wird und daß der Puffer zusätzlich 10 g/l Rinderserumalbumin und 150 mmol/l NaCl enthält.

1.3 Rezeptor $R_2$-Lösung, bindefähig mit TSH:

Als Rezeptor $R_2$ wird ein monoklonaler Maus-anti-TSH-Antikörper der Subklasse $IgG_1$ eingesetzt. Die diesen Antikörper enthaltende Ascitesflüssigkeit wird ad 1,8 M mit Ammoniumsulfat versetzt. Das Präzipitat wird in einem Puffer aus 15 mM Natriumphosphat, pH 7,0 und 50 mM Natriumchlorid aufgenommen. Die so erhaltene Lösung wird einer Passage über DEAE- Cellulose unterworfen. Das so enthaltende, mit TSH-bindefähigen Antikörper enthaltene Eluat, wird mit Puffer II auf eine Protein-Konzentration von 1 $\mu$g/ml verdünnt.

1.4 Rezeptor $R_2'$-Lösung, nicht-bindefähig mit TSH:

Diese Lösung wird analog der Rezeptor $R_2$-Lösung, bindefähig mit TSH (1.3) hergestellt, mit dem Unterschied, daß hier ein monoklonaler Maus-anti-CEA-Antikörper der Subklasse $IgG_1$ eingesetzt wird.

1.5 Markierte Rezeptor $R_1$-Lösung:

Als Rezeptor $R_1$ wird ebenfalls ein monoklonaler Maus-Anti-TSH-Antikörper eingesetzt, der jedoch eine andere antigene Determinante als Rezeptor $R_2$ erkennt. Die diesen Antikörper enthaltende Ascitesflüssigkeit wird, wie unter 1.3 angegeben, aufgereinigt. Der vollständige Antikörper wird in bekannter Weise nach der Methode von R.R. Porter, Biochem. J. 73, (1959) S. 119, zum Fab-Fragment gespalten. Die erhaltenen Fab-Fragmente werden gemäß Ishikawa et al. J. of Immunoassay, 4 (1983) S. 209-327,mit $\beta$-Galactosidase gekoppelt. Die Rezeptor 1-Lösung wird in Puffer II auf eine Konzentration von 500 mU/ml (gemessen mit o-Nitrophenyl-$\beta$-Galactosid bei 37°) verdünnt.

1.6 Rezeptor $R_3$-Lösung:

Schaf-anti-Maus-Fc$\gamma$-Antiserum wird ad 1,8 M mit Ammoniumsulfat versetzt. Das Präzipitat wird in einem Puffer aus 15 mM Natriumphosphat, pH 7,0 und 50 mM Natriumchlorid aufgenommen. Die so erhaltene Lösung wird einer Passage über DEAE-Zellulose unterworfen. Das den spezifischen Antikörper enthaltende Eluat wird in Puffer I auf eine Proteinkonzentration von 50 $\mu$g/ml verdünnt.

1.7 Substratlösung:

| | |
|---|---|
| Chlorphenolrot-$\beta$-galactosid (hergestellt nach DE 33 45 748) | 5 mmol/l (3,05 g/l) |
| HEPES | 70 mmol/l (16,7 g/l) |
| NaCl | 154 mmol/l ( 9 g/l) |
| Rinderserumalbumin | 0,3 % ( 3 g/l) |
| Tween® 20 | 0,2 % ( 2 g/l) |
| pH (mit NaOH) | 7,25 |

2. Durchführung
(Alle Inkubationen erfolgen bei Raumtemperatur)

Mikrotiterplatten (MTP, 96 Vertiefungen pro Platte) aus Polystyrol werden mit 300 $\mu$l Rezeptor $R_3$-Lösung pro Vertiefung 1 Stunde inkubiert. Anschließend wird die Flüssigkeit verworfen. Restliche nicht-spezifische Bindungsstellen werden durch 1/2-stündige Inkubation von 300 $\mu$l Puffer II-Lösung pro Napf abgesättigt. Nachfolgend wird mit je 200 $\mu$l pro Napf die eine Hälfte der Mikrotiterplatte mit Rezeptor $R_2$-Lösung, bindefähig mit TSH (1.3) inkubiert (Teil A der MTP), und die andere Hälfte der Mikrotiterplatte mit Rezeptor $R_2'$-Lösung, nicht bindefähig mit TSH (1.4) für 1 Stunde inkubiert (Teil B der MTP). Nach Verwerfen der Rezeptor-Lösungen in den Näpfen wird jeder Napf zweimal mit 300 $\mu$l Puffer II gewaschen.

Bei der nachfolgenden 18-stündigen Probeninkubation werden jeweils 200 $\mu$l einer Probe in Näpfe gegeben, die den mit TSH bindefähigen Antikörper enthalten (Teil A der MTP) und weitere 200 $\mu$l in Näpfe gegeben, die den nicht mit TSH bindefähigen Antikörper enthalten (Teil B der MTP). Nach Abschluß der Probeninkubation werden die Proben verworfen und die Näpfe dreimal mit je 300 $\mu$l Puffer II gewaschen.

Danach werden sämtliche MTP-Näpfe mit je 200 $\mu$l markierter Rezeptor $R_1$-Lösung (1.5) 3 Stunden inkubiert. Nach Verwerfen der markierten Rezeptor $R_1$-Lösung und dreimaligen Waschen mit je 300 $\mu$l Puffer II wird die Substratreaktion durch Zugabe von 250 $\mu$l Substratlösung (1.7) gestartet. Nach ca. 1 Stunde Inkubationszeit wird die durch Substratumsetzung gebildete Farbe durch Messung bei 570 nm mittels eines handelsüblichen MTP-Photometers erfaßt.

3. Auswertung:

Für jede Probe wird jeweils eine Extinktion aus Teil A der MTP (Extinktion A) und aus Teil B der MTP (Extinktion B) ermittelt. Die Differenz der Extinktionen A und B für jeweils 1 Probe ist auszuwerten. Das so erhaltene, für TSH spezifische Meßsignal wird an Hand einer Eichkurve (siehe unten) einem TSH-Gehalt zugeordnet.

Zur Erstellung der Eichkurve werden für jede MTP Proben mit untersciedlichen, aber bekannten TSH-Gehalten aufgestockt. (Hierfür sind möglichst Proben geringen TSH-Ausgangsgehaltes zu benutzen.) Aus dem durch Aufstockung erhaltenen Signalzuwachs in der jeweiligen Probe und der zur Aufstockung benutzten TSH-Konzentrationen wird eine Eichkurve erstellt, die durch den Nullpunkt des Diagramms extrapoliert wird. An dieser Eichkurve können dann die TSH-Gehalte der unbekannten Proben, unter Berücksichtigung des probenspezifischen Leerwertes, ermittelt werden.

Bei einem typischen Versuch nach dem beschriebenen Verfahren wurden folgende Ergebnisse erhalten:

| Probe | TSH-Zusatz (µU/ml) | Extinktion A (mE)[1] | Extinktion B (mE)[2] | Ext.A-Ext.B (mE) | Δ[3] (mE) | ⌊TSH⌋ (µU/ml) | |
|---|---|---|---|---|---|---|---|
| 1a | -- | 89 | 60 | 29 | -- | 0,023 | |
| 1a | 0,25 | 396 | 59 | 337 | 308 | 0,271 | Eich- |
| 1a | 0,5 | 719 | 58 | 661 | 632 | 0,531 | kurve |
| 1a | 1,0 | 1336 | 63 | 1273 | 1244 | 1,023 | s. Abb. |
| 1b | -- | 155 | 102 | 53 | -- | 0,043 | |
| 1c | -- | 759 | 165 | 594 | -- | 0,477 | |
| 1d | -- | 85 | 49 | 36 | -- | 0,029 | |
| 1e | -- | 201 | 63 | 138 | -- | 0,111 | |
| 1f | -- | 64 | 41 | 23 | -- | 0,018 | |
| 1g | -- | 474 | 110 | 412 | -- | 0,331 | |
| 1h | -- | 110 | 62 | 48 | -- | 0,039 | |
| 1i | -- | 173 | 60 | 113 | -- | 0,091 | |

1) Extinktion bei Verwendung eines mit TSH bindefähigen Antikörpers als Rezeptor $R_2$

2) Extinktion bei Verwendung eines mit TSH nicht bindefähigen Antikörpers als Rezeptor $R_2'$

3) Zunahme von (Ext. A - Ext. B) nach Zugabe von TSH in Probe 1a.

**Beispiel 2**

Bestimmung von $\alpha$-Fetoprotein (AFP) in Humanseren mit monoklonalen Antikörpern

Beispiel 2 entsprecht Beispiel 1 bis auf folgende Unterschiede:

a) Als Rezeptor $R_2$, bindefähig mit AFP, wird ein monoklonaler Maus-anti-AFP-Antikörper der Subklasse $IgG_{2a}$ eingesetzt.

b) Als Rezeptor $R_2'$ , nicht bindefähig mit AFP, wird ein monoklonaler Maus-anti-Ferritin-Antikörper der Subklasse $IgG_{2a}$ eingesetzt.

c) Als Rezeptor $R_1$ wird ein monoklonaler Maus-anti-AFP-Antikörpereingesetzt, der jedoch eine andere antigene Determinante als der Rezeptor $R_2$ erkennt.

d) Die Proben werden vor der Bestimmung 1 : 5 mit Puffer II verdünnt.

e) Die verdünnt Proben werden 1 Stunde in der MTP inkubiert.

f) Die Inkubation mit der markierten Rezeptor $R_1$-Lösung dauert 1 Stunde.

Bei einem typischen Versuch nach dem beschriebenen Verfahren wurde folgende Ergebnisse erhalten:

| Probe | AFP-Zusatz (IU/ml) | Extinktion A (mE) [1] | Extinktion B (mE) [2] | Ext.A-Ext.B (mE) | Δ [3] (mE) | /AFP7 (IU/ml) | |
|-------|------|------|------|------|------|------|------|
| 2a | -- | 219 | 17 | 202 | -- | 1,7 | Eich- |
| 2a | 2 | 456 | 17 | 439 | 237 | 3,7 | kurve |
| 2a | 6 | 918 | 16 | 902 | 700 | 7,7 | s. Abb. 3 |
| | | | | | | | |
| 2b | -- | 208 | 17 | 191 | | 1,6 | |
| 2b | 2 | 465 | 17 | 448 | | 3,8 | |
| 2c | -- | 143 | 18 | 125 | | 1,1 | |
| 2c | 2 | 398 | 19 | 379 | | 3,2 | |
| 2d | -- | 394 | 19 | 375 | | 3,2 | |
| 2e | -- | 512 | 15 | 497 | | 4,2 | |
| 2f | -- | 272 | 18 | 254 | | 2,2 | |
| 2g | -- | 130 | 17 | 113 | | 1,0 | |
| 2h | -- | 152 | 18 | 134 | | 1,1 | |
| 2i | -- | 456 | 17 | 439 | | 3,7 | |
| 2j | -- | 894 | 14 | 880 | | 7,5 | |

[1] Extinktion bei Verwendung eines mit AFP bindefähigen Antikörpers als Rezeptor $R_2$

[2] Extinktion bei Verwendung eines mit AFP nicht bindefähigen Antikörpers als Rezeptor $R_2'$

[3] Zunahme von (Ext. A-Ext. B) nach Zugabe von AFP in Probe 2a).

**Beispiel 3**

Bestimmung von $\alpha$-Fetoprotein (AFP) in Humanseren mit polyklonalen Antikörpern

Beispiel 3 entspricht Beispiel 2 bis auf folgende Unterschiede:

a) Als Rezeptor $R_2$, bindefähig mit AFP, wurde ein polyklonales Kaninchen-anti-AFP-Antiserum eingesetzt.

b) Als Rezeptor $R_2'$, nicht bindefähig mit AFP, wird unspezifisches Kaninschenserum eingesetzt.

c) Als Rezeptor $R_1$ wird ein polyklonales Schaf-anti-AFP-Antiserum eingesetzt, welches jedoch eine andere antigene Determinante als der Rezeptor $R_3$ erkennt. Die Präparation der markierten Rezeptor-1-

8

EP 0 243 655 B1

Lösung erfolgt analog dem Vorgehen bei monoklonalen Antikörpern (s.o.).
d) Als Rezeptor $R_3$ wird ein polyklonales Schaf-anti-Kaninchen-Fc$\gamma$-Antiserum eingesetzt.

Bei einem typischen Versuch nach dem beschriebenen Verfahren wurden folgende Ergebnisse erhalten:

| Probe | AFP Zusatz (IU/ml) | Ext. A (mE) [1] | Ext. B (mE) [2] | Ext. A- Ext. B (mE) | $\triangle$ [3] | [AFP] (IU/ml) |
|---|---|---|---|---|---|---|
| 3a | -- | 260 | 130 | 130 | -- | 1,9 |
| 3a | 2,0 | 403 | 125 | 278 | 148 | 4,1 |
| 3b | -- | 312 | 77 | 235 | -- | 3,4 |
| 3b | 2,0 | 435 | 75 | 360 | 125 | 5,3 |
| 3c | -- | 296 | 150 | 130 | | 1,9 |
| 3d | -- | 328 | 86 | 242 | | 3,5 |
| 3e | -- | 257 | 121 | 136 | | 2,0 |

1)    Extinktion bei Verwendung eines mit AFP bindefähigen *Antikörpers*
      als Rezeptor $R_2$.

2)    Extinktion bei Verwendung eines nicht mit AFP bindefähigen
      Antikörpers als Rezeptor $R_2'$.

3)    Zunahme von (Ext.A-Ext.B) nach Zugabe von AFP in Pro-
      be 3a und 3 b. Die Eichkurvensteilheit wurde aus dem
      Mittelwert von $\triangle$ für Probe 3 a und 3 b ermittelt.

**Beispiel 4**

Bestimmung von TSH

Die verwendeten Rezeptoren und die Substratlösung sind identisch mit den Reagentien aus Beispiel 1, ebenso die Aufreinigungsschritte der Rezeptoren bis einschließlich der Passage über DEAE-Cellulose und die Synthese der markierten Rezeptor-1-Lösung. Mit den beschriebenen Rezeptoren werden Reagenzträger hergestellt.

Herstellung von Reagenzträgern

1) Reagenzträger 1 (bindefähig mit TSH): 40 µl einer Lösung, die pro Liter 100 mmol Natriumphosphat pH 7,3 (37°C), 2 mmol Magnesiumchlorid, 9 g Natriumchlorid, 5 g Rinderserumalbumin, 5 mg Anti-TSH-monoklonale Antikörper aus Maus (Rezeptor $R_2$), 1.000 U Anti-TSH-Antikörper-(Maus)-Fab-Fragment-$\beta$-Galactosidase-Konjugat (Rezeptor-$R_1$-Lösung); Aktivität bestimmt mit ortho-Nitrophenyl-$\beta$-D- galactosid bei 37°C. enthält, wird auf ein Vlies aufgetropft, das aus kommerziellen Polyesterpapier besteht. Anschließend wird bei Raumtemperatur getrocknet. Diese Vliese werden bis zu ihrer Verwendung bei 4°C und einer relativen Luftfeuchtigkeit von 20 % aufbewahrt.
2) Reagenzträger 1' (nicht bindefähig mit TSH): Die Herstellung erfolgt wie für Reagenzträger 1, bis auf den Unterschied, daß anstelle des anti- TSH-Antikörpers (Rezeptor $R_2$) ein anti-CEA-monoklonaler

9

Antikörper aus Maus (Rezeptor $R_2'$) benutzt wird.

3. Reagenzträger 2: Auf ein Zellulose-Vlies werden nach dem Bromcyan- Aktivierungsverfahren (DE-OS 1768512) Schaf-Antikörper gegen den Fc$\gamma$-Teil von Maus-Antikörpern (Rezeptor-$R_3$-Lösung) fixiert, wobei pro g Fasermaterial 10 $\mu$g Antikörper zur Fixierung angeboten werden. Durch Waschen wird ungekoppelter Antikörper entfernt und das Vlies schonend bei Raumtemperatur getrocknet. Die Lagerung der so erhaltenen Vliese folgt analog Reagenzträger 1.

Die Bestimmung mit Hilfe dieser beiden Reagenzträger 1 und 2, bzw. 1' und 2, erfolgt mit der in der DE-AS 3 425 008.5 beschriebenen Vorrichtung zur Durchführung analytischer Bestimmungen (Fig. 2).

Diese lehrt ein Rotoreinsatzelement für Zentrifugalanalysenautomaten, bestehend aus einem Formkörper, der eine Probenauftragskammer, die mit einer Mehrzahl von Reagenzfeldern in Verbindung steht, die jeweils ein mit einem bestimmten Reagenz imprägniertes saugfähiges Trägermaterial enthalten, wenigstens eine Mischventilkammer und eine Meßkammer aufweist, die zusammen einen Probenflüssigkeitstransportweg bilden, der von radial innen nach radial außen führt, wenn das Einsatzelement auf dem Rotor befestigt ist und weiter wenigstens eine weitere Kammer für die Aufnahme einer Flüssigkeit und einen Transportweg, der von dieser Kammer zur Meßführung führt und mit dem Probenflüssigkeitstransportweg mindestens teilweise identisch ist, aufweist. Dabei führt der Probenflüssigkeitstransportweg von einer Porbenauftragskammer (P) über eine mit saugfähigen Material gefüllte, Puffer enthaltende Kammer (a), eine Kammer (c) und eine zwischen den Kammern (a) und (c) angeordnete erste Ventilkammer (Vk1) zu einer zweiten Ventilkammer (Vk2) und von dieser über die Kammer (d) und über eine Auffangkammer (AK) zur Meßkammer (K). Zur Aufnahme einer weiteren Flüssigkeit ist eine als Pumpenkammer ausgebildete Substratkammer (PK) vorgesehen, welche über eine aus Dosierkammer (DK) und Kapillare (Kap) bestehende Dosiereinrichtung und eine Überlaufkammer (ÜK) mit der zweiten Ventilkammer (Vk2) verbunden ist. Fig. 2 zeigt schematisch das verwendete Rotoreinsatzelement.

Zur Bestimmung der Extinktion A (spezifisches Meßsignal einschl. Probenleerwert) werden Reagenzträger 1 und Reagenzträger 2 benutzt, zur Bestimmung der Extinktion B (Probenleerwert) werden Reagenzträger 1' und 2 benutzt.

Reagenzträger 1, bzw. 1', wird auf Feld c des Rotoreinsatzelements plaziert und Reagenzträger 2 auf Feld d. Dabei werden 40 $\mu$l konzentrierter Probe durch eine Öffnung am oberen Rand direkt auf das Feld a pipettiert. 270 $\mu$l Substratlösung werden in Kammer PK pipettiert. Durch ein geeignetes Zentrifugations-Programm, bei dem hohe Drehzahlen mit Stillstand abwechseln, werden dann Probe und Substratlösung in Richtung Trennmatrix und Küvette gefördert.

Dabei werden im Verlauf des Programms die Rezeptoren $R_1$ und $R_2$ bzw. $R_2'$ durch die Probenflüssigkeit vom Feld c eluiert und die homogene Mischung anschließend zur Reaktion gebracht. Auf Feld d werden die gebildeten Komplexe an den Rezeptor $R_3$ gebunden. Der Transfer der Probe von Feld c nach d erfolgt innerhalb sehr kurzer Zeit.

Die Substratlösung wird durch die Dosierkammer DK in Portionen geteilt, von denen die ersten zum Auswaschen von überschüssigem, nicht komplexiertem Konjugat dienen. Die über Komplexbildung auf d gebundene $\beta$-Galactosidase-Aktivität ist proportional zur in der Probe enthaltenen TSH-Menge bzw. zum Probenleerwert. Diese Aktivität wird mit einer weiteren Substratportion bestimmt, wobei das Substrat in einer 5minütigen Reaktion zu farbigen Produkten umgesetzt wird. Die gebildete Farbe und die weitere Farbentwicklung/min in der flüssigen Phase werden in der Küvette bei 576 mm gemessen.

Unter diesen Bedingungen wurden folgende Resultate erhalten:

| Probe | TSH-Zusatz ($\mu$U/ml) | Ext.A[1] (mE) | Ext.B[2] (mE) | Ext. A-Ext. B (mE) | $\Delta$[3] (mE) | [TSH] ($\mu$U/ml) |
|---|---|---|---|---|---|---|
| 4a | -- | 599 | 598 | -1 | -- | 0,0 |
| 4a | 7 | 1602 | 596 | 1006 | 1007 | 6,6 |
| 4a | 13 | 2716 | 641 | 2075 | 2076 | 13,7 |
| 4b | -- | 752 | 766 | -14 | | 0,0 |
| 4c | -- | 2465 | 561 | 1904 | | 12,5 |
| 4d | -- | 689 | 599 | 90 | | 0,6 |
| 4e | -- | 744 | 612 | 132 | | 0,9 |
| 4f | -- | 745 | 515 | 230 | | 1,5 |
| 4g | -- | 650 | 571 | 79 | | 0,5 |
| 4h | -- | 2286 | 558 | 1728 | | 11,4 |

Alle Messungen wurden bei 576 nm bei einer Schichtdicke von 0,3 cm durchgeführt und auf Schichtdicke d = 1 cm umgerechnet.

1) Extinktion bei Verwendung des Reagenzträgers 1 (bindefähig mit TSH)

2) Extinktion bei Verwendung des Reagenzträgers 1' (nicht bindefähig mit TSH).

3) Zunahme von (Ext. A - Ext. B.) nach Zugabe von TSH in Probe 4a.

\* Eichung

## Beispiel 5

Bestimmung von monoklonalen anti-AFP-Antikörpern

Beispiel 5 entspricht Beispiel 1 bis auf folgende Unterschiede:

1. Als Rezeptor-$R_3$-Lösung wird ein Schaf-anti-Kaninchen-Fc$\gamma$-Antiserum verwandt.

2. Als Rezeptor $R_2$, bindefähig mit AFP, wird ein Kaninchen-anti-AFP-Antiserum eingesetzt. Die Aufreinigung des Antikörpers erfolgt wie für Rezeptor $R_3$.

3. Als Rezeptor $R_2'$, nicht bindefähig mit AFP, wird ein unspezifisches Kaninchenserum benutzt. Die Aufreinigung des Antikörpers erfolgt wie für Rezeptor $R_3$.

4. Als Rezeptor $R_1$ wird ein Schaf-anti-Maus-IgG-Antikörper eingesetzt. Die Aufreinigung erfolgt wie für Rezeptor $R_3$. Der Antikörper wird nach der Methode von Nakane (M.B. Wilson, P.K. Nakane "Recent Developments in the Periodate Method of Conjugating. Horseradish Peroxidase to Antibodies", 1978, Elsevier, North Holland Biomedical Press p. 215-224 in "Immunofluorescence and Related Staining Techniques") mit Peroxidase aus Meerrettich gekoppelt. Das Antikörper-Enzym-Konjugat wird in Puffer II auf eine Koinzentratin von 80 U/l (gemessen mit Guajacol und $H_2O_2$ als Substrat bei 25°C) eingestellt.

5. Nach Inkubation der Mikrotiterplatte mit Rezeptor $R_2$ bzw. Rezeptor $R_2'$ und Waschung entsprechend Beispiel 1 wird jeder Napf 1 h mit 200 $\mu$l einer Lösung von 12,5 IU/ml AFP in Puffer II inkubiert. Anschließend wird zweimal mit 300 $\mu$l Puffer II gewaschen.

6. Die nachfolgende Inkubation mit Antikörper-haltigen Probelösungen beträgt 1 h.

7. Die Inkubation mit markierter Rezeptor-1-Lösung dauert 1 h.

8. Als Indikatorlösung wird verwendet:

1,8 mM ABTS[R] (2,2'-Azino-di-[3-ethylbenzthiazoliumsulfonat(6)]) 3,3 mM Natriumperborat in 100 mM Phosphat-Citrat-Puffer, pH 4,4. Die Inkubation dauert 1 h. Die Messung der gebildeten Farbe erfolgt bei 405 nm nach Abgleich des Photometers gegen den Substratleerwert.

Bei einem typischen Versuch nach dem beschriebenen Verfahren wurden Puffer-II-Lösungen mit unterschiedlichen Antikörpern in wechselnden Konzentrationen versetzt. Dabei wurden folgende Ergebnisse erhalten:

| monoklonaler Maus-Antikörper-Zusatz | | Ext. A | Ext. B | Ext. A- Ext. B |
|---|---|---|---|---|
| Art | Menge (µg/ml) | (mE) | (mE) | (mE) |
| - | - | 258 | 267 | - 9 |
| anti-AFP - 1* | 0,01 | 442 | 272 | 170 |
| anti-AFP - 1 | 0,03 | 518 | 251 | 267 |
| anti-AFP - 1 | 0,10 | 589 | 261 | 328 |
| anti-AFP - 2* | 0,01 | 321 | 245 | 76 |
| anti-AFP - 2 | 0,03 | 416 | 245 | 171 |
| anti-AFP - 2 | 0,10 | 471 | 244 | 227 |
| anti-Ferritin | 1,00 | 228 | 248 | -20 |
| anti-CEA | 1,00 | 254 | 257 | - 3 |

\* 2 unterschiedliche monoklonale anti-AFP-Antikörper

## Patentansprüche

1. Verfahren zur Bestimmung eines Reaktionspartners einer immunologischen Reaktion nach dem Prinzip des Immunoassays, wobei der zu bestimmende Reaktionspartner mit einem markierten spezifischen Rezeptor $R_1$, und mindestens einem unmarkierten Rezeptor $R_2$ in Kontakt gebracht wird und wobei einer der unmarkierten Rezeptoren $R_2$ an eine Festphase über eine bindefläche Substanz $R_3$ gebunden ist , **dadurch gekennzeichnet,** daß zur Bestimmung des Probenleerwertes der unmarkierte Rezeptor

$R_2$, welcher von dem an die Festphase fixierten Rezeptor gebunden wird, durch einen anderen unmarkierten Rezeptor $R_2'$, der mit dem anderen Reaktionspartner von $R_2$ nicht reagiert, ersetzt wird.

2. Verfahren nach Anspruch 1, **dadurch gekennzeichnet,** daß der unmarkierte Rezeptor $R_2$ gegen den zu bestimmenden Reaktionspartner oder dessen Komplex mit Rezeptor $R_1$ gerichtet ist.

3. Verfahren nach Anspruch 1 oder 2, **dadurch gekennzeichnet,** daß als bindefähige Substanz $R_3$ ein Anti-Ig-Antikörper verwendet wird, welcher $R_1$ nicht bindet.

4. Verfahren nach einem der Ansprüche 1 bis 3, **dadurch gekennzeichnet,** daß die Rezeptoren $R_2$ und $R_2'$ monoklonal sind und der gleichen Subklasse angehören.

5. Verfahren nach einem der Ansprüche 1 bis 4, **dadurch gekennzeichnet,** daß als Rezeptoren $R_2$ und $R_2'$ haptenisierte Antikörper und als bindefähige Substanz $R_3$ ein gegen das Hapten gerichteter Antikörper verwendet werden.

6. Reagenz zur Bestimmung eines Reaktionspartners einer immunologischen Reaktion **dadurch gekennzeichnet,** daß es eine an eine Festphase gebundene bindefähige Substanz $R_3$, mindestens einen mit $R_3$ und einem weiteren Reaktionspartner bindefähigen unmarkierten Rezeptor $R_2$ und einen markierten spezifischen Rezeptor $R_1$ enthält und physikalisch getrennt davon eine weitere Menge der an eine Festphase gebundenen bindefähigen Substanz $R_3$, einen unmarkierten Rezeptor $R_2'$, der mit dem anderen Reaktionspartner von $R_2$ nicht reagiert und eine weitere Menge an markiertem spezifischen Rezeptor $R_1$ enthält.

7. Reagenz nach Anspruch 6, **dadurch gekennzeichnet,** daß die Rezeptoren $R_2$ bzw. $R_2'$ an die Festphase über einen Anti-Ig-Antikörper gebunden vorliegen.

8. Reagenz nach Anspruch 6, **dadurch gekennzeichnet,** daß die Rezeptoren $R_2$ bzw. $R_2'$ haptenisiert sind und an die Festphase über einen gegen das Hapten gerichteten Antikörper gebunden ist.

**Claims**

1. A process for the determination of a reaction partner in an immunological reaction in accordance with the principle of immunoassays, in which the reaction partner to be determined is brought into contact with a marked specific receptor $R_1$ and at least one unmarked receptor $R_2$ and in which one of the unmarked receptors $R_2$ is bonded on a solid phase by means of a substance $R_3$ with binding property, wherein for the determination of the sample blank value, the unmarked receptor $R_2$, which is bonded by the receptor fixed on the solid phase, is replaced by another unmarked receptor $R'_2$, which does not react with the other reaction partner of $R_2$.

2. Process as set forth in claim 1 wherein said unmarked receptor $R_2$ is oriented against the reaction partner to be determined or its complex with receptor $R_1$.

3. Process as set forth in claim 1 or 2, wherein as the substance $R_3$ with binding property, an anti-Ig-antibody is used, which does not bind $R_1$.

4. Process as set forth in one of the claims 1 to 3, wherein the receptors $R_2$ and $R'_2$ are monoclonal and belong to the same sub-class.

5. Process as set forth in one of the claims 1 to 4, wherein as the receptors $R_2$ and $R'_2$ haptenized antibodies and as the substance $R_3$ with binding property, an antibody directed against the haptene are used.

6. Reagent for the determination of a reaction partner in an immunological reaction wherein said reagent contains a substance $R_3$ with binding property which is bonded on a solid phase, at least one unmarked receptor $R_2$ capable of binding with $R_3$ and a further reaction partner, and also contains a marked specific receptor $R_1$, and contains physically separated therefrom a further amount of the substance $R_3$ capable of binding and bonded on the solid phase, an unmarked receptor $R'_2$, which

EP 0 243 655 B1

does not react with the other reaction partner of $R_2$ and a further amount of the marked specific receptor $R_1$.

7. Reagent as set forth in claim 6, wherein the receptors $R_2$ or $R'_2$ are present bonded to the solid phase by an anti-Ig-antibody.

8. Reagent as set forth in claim 6, wherein said receptors $R_2$ and/or $R'_2$ are haptenized and are bonded to the solid phase by an antibody directed against the haptene.

## Revendications

1. Procédé de détermination d'un partenaire réactionnel d'une réaction immunologique selon le principe des déterminations immunologiques, dans lequel le partenaire réactionnel à déterminer est mis en contact avec un récepteur spécifique marqué $R_1$ et avec au moins un récepteur non marqué $R_2$ et dans lequel l'un des récepteurs non marqués $R_2$ est lié à une phase solide par l'intermédiaire d'une substance capable de liaison $R_3$, caractérisé en ce que, pour la détermination de la valeur zéro de l'échantillon, le récepteur non marqué $R_2$, qui est lié au récepteur fixé à la phase solide, est remplacé par un autre récepteur non marqué $R_2'$ qui ne réagit pas avec l'autre partenaire réactionnel de $R_2$.

2. Procédé selon la revendication 1, caractérisé en ce que le récepteur non marqué $R_2$ est dirigé conte le partenaire réactionnel à déterminer ou contre son complexe avec le récepteur $R_1$.

3. Procédé selon la revendication 1 ou 2, caractérisé en ce que l'on utilise conme substance capable de liaison $R_3$ un anticorps anti Ig qui ne se lie pas à $R_1$.

4. Procédé selon l'une des revendications 1 à 3, caractérisé en ce que les récepteurs $R_2$ et $R_2'$ sont monoclonaux et appartiennent à la même sous-classe.

5. Procédé selon l'une des revendications 1 à 4, caractérisé en ce que l'on utilise comme récepteurs $R_2$ et $R_2'$ des anticorps hapténisés et comme substance capable de liaison $R_3$ un anticorps dirigé contre l'haptène.

6. Réactif pour déterminer un partenaire réactionnel d'une réaction immunologique, caractérisé en ce qu'il contient une substance capable de liaison $R_3$ liée à une phase solide, au moins un récepteur non marqué $R_2$ capable de liaison avec $R_3$ et avec un autre partenaire réactionnel et un récepteur spécifique marqué $R_1$ et, séparés physiquement de ceux-ci, une autre quantité de substance capable de liaison $R_3$ liée à une phase solide, un récepteur non marqué $R_2'$ qui ne réagit pas avec l'autre partenaire réactionnel de $R_2$ et une autre quantité de récepteur spécifique marqué $R_1$.

7. Réactif selon la revendication 6, caractérisé en ce que les récepteurs $R_2$ ou $R_2'$ sont liés à la phase solide par l'intermédiaire d'un anticorps anti Ig.

8. Réactif selon la revendication 6, caractérisé en ce que les récepteurs $R_2$ ou $R_2'$ sont hapténisés et sont liés à la phase solide par l'intermédiaire d'un anticorps dirigé contre l'haptène.

14